# EUROPEAN PATENT SPECIFICATION

(11) **EP 1 578 325 B1**
(45) Date of publication and mention of the grant of the patent: **27.04.2011**
(21) Application number: 03815011.6
(22) Date of filing: 31.12.2003
(51) Int. Cl.: A61K 47/10, A61K 47/24, A61K 38/28, A61K 9/00

(54) **STABLE TOPICAL DRUG DELIVERY COMPOSITIONS**
STABILE ZUSAMMENSETZUNGEN ZUR TOPISCHEN ARZNEIMITTELABGABE
COMPOSITIONS STABLES POUR UNE ADMINISTRATION DE MEDICAMENT TOPIQUE

(30) Priority: 31.12.2002 US 437279 P
(43) Date of publication of application: 28.09.2005
(73) Proprietor: Transdermal Biotechnology, Inc., Meriden, CT 06450 (US)
(72) Inventor: PERRICONE, Nicholas, V., Meriden, CT 06450 (US); POTINI, Chim, Blommington, IL 61704 (US)
(74) Representative: Maiwald Patentanwalts GmbH
(86) International application number: PCT/US2003/041671
(87) International publication number: WO 2004/060315

(56) References cited:
- WO-A1-01/49268
- WO-A1-02/064115
- WO-A1-02/064166
- CA-A1- 2 181 390
- US-A- 5 858 398
- US-A- 6 165 500

## Description

### Field Of The Invention

The present invention relates to topical drug delivery compositions and methods of transdermal drug delivery. More specifically, the present invention relates to stable drug delivery compositions for topical administration.

### Background Of The Invention

Topical drug delivery systems are known. These systems deliver drugs, therapeutic agents and other desired substances transdermally and may be designed to act locally at the point of application or to act systemically once entering the body's blood circulation. In these systems, delivery may be achieved by means such as direct topical application of a substance or drug in the form of an ointment or the like, or by adhesion of a patch with a reservoir or the like that holds the drug and releases it to the skin in a time-controlled fashion.

Transdermal delivery systems for agents such as drugs, pain relieving compounds, vitamins, and skin improving compounds has been in use for a number of years. These transdermal delivery systems using creams have been developed for use with analgesics and skin refining compounds. Transdermal systems using a patch have been developed for nicotine and estrogen therapies, for instance, estradiol technology described in U.S. Patent No. 6,521,250 to Meconi, et al.

While effective for their purpose, these systems have typically only been useful for transdermal delivery of relatively small molecules. The skin's porous structure permits such small molecules to pass from the epidermis to the dermis via diffusion. However, large molecules, such as insulin, are not able to diffuse through the skin and cannot be delivered by these known means.

While the delivery of large molecules such as insulin have been addressed, such systems do not address the storage and retention of the effectiveness of the drug to be delivered. Many pharmaceuticals and biologically active compounds, such as insulin, must be kept cool and away from heat to remain effective and prevent denaturing at ambient temperatures. Such substances may not be stored or carried (without refrigeration) by the user. Often drugs like insulin must be administered throughout the day and should be in ready-access to or carried by the user, which may expose the compound to high temperatures. As such, there remains a need to stabilize compositions, including insulin, so that they are resistant to warmer temperatures and have a longer life at these temperatures without a need for refrigeration

### Summary Of The Invention

A composition for transdermal delivery of a macromolecule comprises a phosphatidylcholine carrier component entrapping the macromolecule, wherein the carrier component stabilizes the macromolecule at room temperature.

A method for administering a drug comprises applying to skin composition containing an effective amount of the drug, a carrier having a phosphatidylcholine component entrapping the drug.

### Detailed Description Of The Invention

Phosphatidylcholine is used as a carrier for the topical drug delivery of polypeptides and macromolecules in the practice of this invention. Phosphatidylcholine is a basic component of cell membrane bilayers and the main phospholipid circulating in the plasma. Phosphatidylcholine is highly absorbable and supplies choline which is needed to facilitate movement of fats and oils across and maintain cell membranes in animals.

Topical drug delivery compositions of the present invention are non-polar and formulated to contain polypeptides and macromolecules soluble in phosphatidylcholine, which are then applied to skin for transdermal delivery of the macromolecule. Topical drug delivery compositions of the invention are efficacious in the delivery of macromolecular drugs that are conventionally administered intramuscularly, intravenously or orally, including, but not limited to polypeptides such as insulin and somatropin, prostaglandins, glucocorticoids, estrogens, androgens, and the like.

It is an advantage of the invention that topical administration of a composition and transdermal delivery of the drug therein is easier and pleasanter as an administration route than injections, particularly for drugs such as insulin that must be given to patients over a period of time, or for a lifetime. Furthermore, unlike oral administration where a substantial amount of the drug can be destroyed in the digestive process, the drugs in a topical application are not wasted. Topical application allows a steady diffusion of the drug to the desired target area without the cyclic dosages typical of orally or parenterally administered drugs.

The term "phosphatidylcholine" as used herein means a mixture of stearic, palmitic, and oleic acid diglycerides linked to the choline ester of phosphoric acid, commonly called lecithin. Many commercial lecithin products are available, such as, for example, Lecithol®, Vitellin®, Kelecin®, and Granulestin® because lecithin is widely used in the food industry. Compositions of the invention can contain synthetic or natural lecithin, or mixtures thereof. Natural preparations are preferred because they exhibit desirable physical characteristics and are both economical and nontoxic.

Preferred topical drug delivery compositions of the present invention additionally contain polyenylphosphatidylcholine (herein abbreviated "PPC") to enhance epidermal penetration. The term "polyenylphosphatidylcholine" as used herein means any phosphatidylcholine bearing two fatty acid substituents, wherein at least one is an unsaturated fatty acid with at least two double bonds such as linoleic acid. Certain types of soybean lecithin and soybean fractions, for example, contain higher levels of polyenylphosphatidylcholine, with dilinoleoylphosphatidylcholine (18:2-18:2 phosphatidylcholine) as the most abundant phosphatidylcholine species, than conventional food grade lecithin, and are useful in formulating topical drug delivery compositions of the invention. Alternatively, conventional soybean lecithin is enriched with polyenylphosphatidylcholine by adding soybean extracts containing high levels of polyenylphosphatidylcholine. As used herein, this type of phosphatidylcholine is called "polyenylphosphatidylcholine-enriched" phosphatidylcholine (hereinafter referred to as PPC-enriched phosphatidylcholine), even where the term encompasses lecithin obtained from natural sources exhibiting polyenylphosphatidylcholine levels higher than ordinary soybean varieties. These products are commercially available from American Lecithin Company, Rhône-Poulenc and other lecithin vendors. American Lecithin Company markets its products with a "U" designation, indicating high levels of unsaturation; Rhône-Poulenc's product is a soybean extract containing about 42% dilinoleoylphosphatidylcholine and about 24% palmitoyllinoleylphosphatidylcholine (16:0-18:2 PC) as the major phosphatidylcholine components.

While not wishing to be bound to any theory, it is believed that the PPC-enriched phosphatidylcholine forms a bilayer enveloping the polypeptide or macromolecule to create the topical drug delivery composition, contributing to the stability of the active molecule and enhancing penetration. Further, the topical drug delivery composition may be in liquid crystal phase, with the PPC-enriched phosphatidylcholine loosely arranged in multilamellar fashion, with the polypeptide or macromolecule being bonded and entrapped within the lipid bilayers formed therein, as disclosed in U.S. Patent Application No. 10/448,632 to Perricone. This forms a loosely arranged, yet stable, PPC-enriched phosphatidylcholine-drug complex that further increases penetration and delivery of the polypeptide or macromolecule to the dermal vasculature.

Topical drug delivery compositions of the present invention provide an administration route that is a marked improvement over conventional insulin injections, considerably easier and pleasanter. It is a further advantage that compositions of the invention are also stable at room temperature, providing considerable convenience for insulin users who, in the past, have had to deal with the refrigerated insulin products commercially available. Also, insulin compositions according to the present invention have longer shelf lives (whether stored at room temperature or refrigerated) and will not denature at room temperature as would traditional insulin treatments.

Insulin useful in the topical drug delivery compositions of the present invention is commercially available from a variety of sources, marketed under the tradenames Humulin®, Novolin®, Humalog®, Inutral®, among others. Some of these products contain porcine sequences. Compositions of the invention are preferably formulated with recombinant human polypeptides such as those obtained from Sigma Co., Spectrum Chemicals and Laboratories, and scimitar vendors and employed in the examples that follow. It is an advantage of the invention that topical drug delivery compositions carrying insulin are formulated with commercially available ingredients.

Topical drug delivery compositions are generally formulated with a carrier comprising a PPC-enriched phosphatidylcholine material with the trade name NAT 8729 (commercially available from vendors such as Rhône-Poulenc and American Lecithin Company and at least one polyglycol (polyhydric alcohol of a monomeric glycol such as Polyethylene glycol (PEG) 200, 300, 400, 600,1000,1450, 3350, 4000, 6000, 8000 and 20000). Further, this carrier may comprise a surfactant such as a siloxylated polyether comprising dimethyl, methyl(propylpolyethylene oxide propylene oxide, acetate) siloxane commercially available from vendors such as Dow Corning (Dow Corning 190 surfactant) and lubricant such as silicone fluids containing low viscosity polydimethylsiloxane polymers, methylparaben (p-hydroxy benzoic acid methyl ester) commercially available from vendors such as Down Corning (Dow Corning 200 silicone fluid). Additionally, purified water may be added to the carrier. The carrier is then mixed with a preparation of the particular polypeptide(s) or macromolecule(s) in an amount to obtain the desired strength in the final composition. The following examples are presented to further illustrate and explain the present invention and should not be taken as limiting in any regard.

### Preparation of Stable Insulin Compositions: Example 1

Stable insulin topical preparations were formulated by first preparing a base solution. A polyenylphosphatidylcholine material denoted NAT 8729 which contained 80.6% PPC-enriched phosphatidylcholine and 4.9% lysophosphatidylcholine was obtained from Rhône-Poulenc. NAT 8729 (45% w/w) was shaved and added to a mixture of polyglycol E200 (50% w/w) and polyglycol E400 (5% w/w) both obtained from Dow Coming. The base solution was then covered well and lightning mixed with a special disintegration head impeller slowly at 800 rpm with slight heat. The temperature did not go above 40°C. Typical mixing times were 5 hours. The final solution is a crystal clear, viscous amber solution with no sediments or separations.

Into this base solution (97.25% w/w) was then mixed a Dow Corning Fluid 190 (1.00% w/w) [a siloxylated polyether comprising dimethyl, methyl(propylpolyethylene oxide propylene oxide, acetate) siloxane]; a Dow Corning silicone fluid denoted 200-5 or 10cst (1.00% w/w) [silicone fluids containing low viscosity polydimethylsiloxane polymers]; and methylparaben [p-hydroxy benzoic acid methyl ester] obtained from Mallinckrodt (0.75% w/w). The ingredients were homogenized with 3850 rpm with a 0.45 micron screen as follows. The methylparaben was first added to the base solution and mixed for at least an hour until a complete solution formed. Then the Dow Corning 200-5 or 10cst was slowly added and mixed until a clear solution formed. Afterwards the Dow Corning Fluid 190 was added slowly and mixed into the solution to form the carrier.

Insulin preparations of the invention were then made using the carrier in two strengths: 50 units and 100 units, by simply dissolving RNA-derived recombinant human insulin obtained from Sigma into the carrier. It was readily soluble in the carrier.

In testing the stability of the stable insulin composition, insulin standards were prepared at 1 mg/ml in 0.01 N HCl using Sigma insulin. (One mg of this material exhibits an activity of 28 insulin units.) Stable insulin compositions samples were prepared at 1 mg/1 ml base by mixing at room temperature for 60 minutes. This mixture was then divided in half, half of which was stored at 4°C, and the other half stored at room temperature. Separation analyses, High Performance Liquid Chromatography (RP-HPLC) and High Performance Capillary Electrophoresis (HPCE), of insulin standards and insulin compositions of the invention which were stored at different temperatures for different periods of time were performed.

The RP-HPLC and HPCE analyses indicated that insulin standards that were stored at 4°C or -20°C were stable after 65 days, but insulin standards stored at room temperature started to denature within 7 days. The RP-HPLC and HPCE profiles of insulin compositions of the invention, on the other hand, were stable at both room temperature and at 4°C, and did not change after 65 days. The results clearly showed that the carrier prevented the denaturing of the insulin stored at room temperature.

### Preparation of Stable Insulin Compositions: Example 2

Stable insulin compositions were formulated by first preparing a base solution. Polyglycol E200 (PEG-200) (50% w/w) was weighed and polyglycol E400 (PEG-400) (5% w/w) was added to the same container to obtain the desired weight, (both obtained from Dow Coming). PEG-200 and PEG-400 were lightning mixed at 38-40° C with IKA model RW20 using a disintegration head impeller slowly at 800 rpm (speed 1), yielding PEG-200/PEG-400 solution. A PPC-enriched phosphatidylcholine material denoted NAT 8729 containing 80.6% PPC-enriched phosphatidylcholine and 4.9% lysophosphatidylcholine was obtained from Rhône-Poulenc. NAT 8729 (45% w/w) was shaved and added to PEG-200/PEG-400 solution, covered and mixed, with temperature not exceeding 40°C, until a clear, viscous amber solution with no sediments or separations resulted. The mixing time was approximately five hours. An alternative mixture can be prepared by covering and mixing the solution overnight without heat for a 95-96% yield. The solution was removed from heat and transferred to Ross Homogenizer (Model HSM100LC) using smallest mesh screen.

A Dow Corning Fluid was then prepared. Dow Corning Fluid denominated 190 (1.00% w/w) [a siloxylated polyether comprising dimethyl, methyl(propylpolyethylene oxide propylene oxide, acetate) siloxane] and Dow Corning Fluid denoted 200-5 or 10 cst (1.00% w/w) [silicone fluids containing low viscosity polydimethylsiloxane polymers] were mixed together in a container with a clean spatula.

The solution (53.25% w/w) was warmed to 40°C and mixed at 800 rpm. Typical mixing times were approximately 5 hours. The solution was then milled at 3800 rpm and the Dow Corning Fluid mixture was added very slowly until the a clear solution resulted. Methyl Paraben (p-hydroxy benzoic acid methyl ester) obtained from Mallinckrodt (0.75% w/w) was added at once and mixed until a complete solution resulted. Purified water warmed to 40°C was added very slowly to solution while milling at 7500 rpm for about three minutes.
At end of milling, speed was increased to 10,000 rpm for few seconds before stopping. The solution was removed and swept with paddle head using IKA Model RW-20 until cooled to room temperature. This step is very critical and if it
is not done properly it will generate a biphasic end product. The general rule is to use a container having a volume twice that of the solution so the homogenizer head is well embedded in the solution. The solution was then cooled to room temperature.

USP human recombinant insulin in obtained from Spectrum Chemicals and Laboratories (Product #11247) was prepared in 0.01 N HCl at 50 mg/ml, and gently, yet well mixed. This insulin preparation was then added very slowly to the above solution to obtain a final concentration of 500 units/ml or 20 mg/ml. Mixing was continued at room temperature for at least one hour. The final stable insulin composition was stored at 4°C in amber air-tight container.

RP-HPLC and HPCE analyses of insulin standards (prepared at 5 mg/ml in 0.01 N HCl) and stable insulin compositions of the invention which were stored at different temperatures for different periods of time were performed.
The results indicated that standard insulin standards stored at 4° were stable up to 22 weeks and started to denature after 34 weeks, whereas when stored at room temperature started to denature within only 1 week. However, the stable insulin compositions prepared in accordance with the above disclosures that were stored at room temperature were stable up to at least 22 weeks, which is 21 weeks longer than the standard. The results showed no change in shelf-life from the standard for stable insulin compositions stored at 4°C (no change after 34 weeks).

Stable topical drug delivery compositions of the present invention may be employed to deliver and stabilize polypeptides transdermally, including but not limited to insulin, oxytocin, vasopressin, insulin, somatotropin, calcitonin, chorionic gonadotropin, menotropins, follitropins, somatostatins, progestins, and combinations of any of these. These drugs are readily available from a variety of commercial sources. Somatotropin (pituitary growth hormone) is marketed under the tradenames Gentropin®, Humatrope®, Nutropin®, and Serostim®.

A drug delivery composition formulated with somatotropin was formulated in one trial with 85% phosphatidylcholine to which lipoic acid and ascorbyl palmitate were added. Somatotropin readily dispersed in phosphatidylcholine and remained stable in it. Growth hormone appeared to penetrate the skin well when the composition was topically applied.

The present invention may also be used to provide topical delivery of active agents other that drugs, for example, skin care agents. The invention is particularly useful with large molecules that are used in some cosmetic formulations, including peptides and polymers.

## Claims

1. A stable transdermal insulin composition comprising a phosphatidylcholine component, said phosphatidylcholine comprising polyenylphosphatidylcholine-enriched phosphatidylcholine and at least one polyglycol, and entrapping said insulin for transdermal delivery to dermal vasculature, wherein said phosphatidylcholine component stabilizes the insulin at room temperature.

2. The transdermal composition of claim 1, wherein said polyglycol comprises a polyglycol having a molecular weight of 200 and a polyglycol having a molecular weight of 400.

3. The transdermal composition of claim 1 or 2, wherein said phosphatidylcholine component comprises 45% w/w phosphatidylcholine, 50% w/w polyglycol having a molecular weight of 200 and 5% w/w polyglycol having a molecular weight of 400.

4. The transdermal composition of any one of claims 1 to 3, further comprising a surfactant, and a lubricant, and methyl paraben.

5. The transdermal composition of claim 4, wherein
said surfactant is a siloxylated polyether;
said lubricant is silicone fluid.

6. The transdermal composition of claim 5, wherein
said siloxylated polyether is dimethyl, methyl(propylpolyethylene oxide propylene oxide, acetate) siloxane; and
said silicone fluid contains low viscosity polydimethylsiloxane polymers.

7. The transdermal composition of any one of claims 1 to 6, wherein said insulin is human recombinant insulin prepared in 0.01 N HCl at 50 mg/ml to obtain an insulin concentration of 20 mg/ml in the stable transdermal insulin composition.

8. A method of formulating a stable transdermal insulin composition for transdermal delivery of insulin to dermal vasculature comprising:
preparing a carrier from a phosphatidylcholine component, said phosphatidylcholine component comprising polyenylphosphatidylcholine-enriched phosphatidylcholine and at least one polyglycol, and
entrapping insulin in the carrier such that the carrier stabilizes the insulin at room temperature.

9. The method of claim 8, wherein preparing said carrier comprises:
combining a polyglycol having a molecular weight of 200 and a polyglycol having a molecular weight of 400 to form a polyglycol mixture;
shaving said phosphatidylcholine component into said polyglycol mixture to form a phosphatidylcholine mixture; and
mixing said phosphatidylcholine mixture until said phosphatidylcholine mixture is clear.

10. The method of claim 8 or 9, wherein preparing said carrier further comprises warming said phosphatidylcholine mixture to 40°C and milling said warmed solution; combining a surfactant and a lubricant to form a fluid;
adding said fluid to said warmed phosphatidylcholine mixture and milling until said solution is clear;
adding methyl paraben to said phosphatidylcholine mixture and milling until said methyl paraben dissolves in said solution;
warming water to 40°C and adding said warmed water slowly to said solution; and ceasing milling of said solution and sweeping said solution to cool to room temperature.

11. The method of any one of claims 8 to 10, wherein said insulin is human recombinant insulin prepared in a solution of 0.01 N HCl at 50 mg/ml and said insulin solution is mixed into said carrier at room temperature for at least one hour.

12. The method of any one of claims 8 to 11, wherein said stable transdermal insulin composition contains insulin at a concentration of 20 mg/ml.

## Patentansprüche

1. Stabile, transdermale Insulinzusammensetzung umfassend einen Phosphatidylcholinbestandteil, wobei das Phosphatidylcholin mit Polyenylphosphatidylcholin angereichertes Phosphatidylcholin und zumindest ein Polyglykol umfasst und das Insulin für die transdermale Abgabe an das dermale Gefäßsystem einschließt, wobei der Phosphatidylcholinbestandteil das Insulin bei Raumtemperatur stabilisiert.

2. Transdermale Zusammensetzung gemäß Anspruch 1, wobei das Polyglykol ein Polyglykol mit einem Molekulargewicht von 200 und ein Polyglykol mit einem Molekulargewicht von 400 umfasst.

3. Transdermale Zusammensetzung gemäß Anspruch 1 oder 2, wobei der Phosphatidylcholinbestandteil 45 Gew.-% Phosphatidylcholin, 50 Gew.-% Polyglykol mit einem Molekulargewicht von 200 und 5 Gew.-% Polyglykol mit einem Molekulargewicht von 400 umfasst.

4. Transdermale Zusammensetzung gemäß einem der Ansprüche 1-3, ferner umfassend einen oberflächenaktiven Stoff, und einen Schmierstoff und Methylparaben.

5. Transdermale Zusammensetzung gemäß Anspruch 4, wobei der oberflächenaktive Stoff ein siloxylierter Polyether und der Schmierstoff eine Silikonflüssigkeit ist.

6. Transdermale Zusammensetzung gemäß Anspruch 5, wobei der siloxylierte Polyether Dimethyl, Methyl(propylpolyethylenoxid propylenoxid, actetat)siloxan ist; und die Silikonflüssigkeit Polydimethylsiloxanpolymere mit niedriger Viskosität enthält.

7. Transdermale Zusammensetzung gemäß einem der Ansprüche 1-6, wobei das Insulin rekombinantes Humaninsulin vorbereitet in 0.01 N HCl mit 50 mg/mL ist, um eine Insulinkonzentration von 20 mg/mL in der stabilen, transdermalen Insulinzusammensetzung zu erhalten.

8. Verfahren zur Herstellung einer stabilen, transdermalen Insulinzusammensetzung für die transdermale Abgabe von Insulin an das dermale Gefäßsystem, umfassend:
Herstellung eines Trägers aus einem Phosphatidylcholinbestandteil, wobei der Phosphatidylcholinbestandteil mit Polyenylphosphatidylcholin angereichertes Phosphatidylcholin und zumindest ein Polyglykol umfasst, und
Einschließen des Insulins in den Träger, dass der Träger das Insulin bei Raumtemperatur stabilisiert.

9. Verfahren gemäß Anspruch 8, wobei die Herstellung des Trägers umfasst:
Kombinieren eines Polyglykols mit einem Molekulargewicht von 200 und eines Polyglykols mit einem Molekulargewicht von 400 um eine Polyglykolmischung zu erhalten;
Einschaben ("shaving") des Phosphatidylcholinbestandteils in die Polyglykolmischung, so dass eine Phosphatidylcholinmischung gebildet wird; und
Mischen der Phosphatidylcholinmischung bis die Phosphatidylcholinmischung klar wird.

10. Verfahren gemäß Anspruch 8 oder 9, wobei die Herstellung des Trägers ferner umfasst:
Erwärmen der Phosphatidylcholinmischung auf 40 °C und das Rühren ("milling") der erwärmten Lösung;
Zugeben eines oberflächenaktiven Stoffes und eines Schmiermittels um eine Flüssigkeit zu bilden;
Zugeben der Flüssigkeit zu der erwärmten Phosphatidylcholinmischung und Rühren bis die Lösung klar wird;
Zugeben von Methylparaben zu der Phosphatidylcholinmischung und Rühren bis sich das Methylparaben in der Lösung löst;
Erwärmen von Wasser auf 40 °C und langsames Zugeben des erwärmten Wassers zu der Lösung; und
Beenden des Rührens der Lösung und Bewegen der Lösung um auf Raumtemperatur abzukühlen.

11. Verfahren gemäß einem der Ansprüche 8-10, wobei das Insulin rekombinantes Humaninsulin vorbereitet in 0.01 N HCl mit 50 mg/mL ist und die Insulinlösung bei Raumtemperatur mindestens eine Stunde in den Träger eingemischt wird.

12. Verfahren gemäß einem der Ansprüche 8-11, wobei die stabile, transdermale Insulinzusammensetzung Insulin mit einer Konzentration von 20 mg/mL enthält.

## Revendications

1. Composition d'insuline transdermique stable comprenant un composant phosphatidylcholine, ladite phosphatidylcholine comprenant une phosphatidylcholine enrichie en polyénylphosphatidylcholine et au moins un polyglycol, et piégeant ladite insuline pour une administration transdermique dans une vasculature dermique, dans laquelle ledit composant phosphatidylcholine stabilise l'insuline à température ambiante.

2. Composition transdermique selon la revendication 1, dans laquelle ledit polyglycol comprend un polyglycol ayant une masse moléculaire de 200 et un polyglycol ayant une masse moléculaire de 400.

3. Composition transdermique selon la revendication 1 ou 2, dans laquelle ledit composant phosphatidylcholine comprend 45% m/m de phosphatidylcholine, 50 % m/m d'un polyglycol ayant une masse moléculaire de 200 et 5 % m/m d'un polyglycol ayant une masse moléculaire de 400.

4. Composition transdermique selon l'une quelconque des revendications 1 à 3, comprenant en outre un agent tensioactif, un lubrifiant et du méthylparabène.

5. Composition transdermique selon la revendication 4, dans laquelle ledit agent tensioactif est un polyéther siloxylé ;
ledit lubrifiant est un fluide de silicone.

6. Composition transdermique selon la revendication 5, dans laquelle
ledit polyéther siloxylé est le diméthyl, méthyl (oxyde de propylpolyéthylène oxyde de propylène, acétate) siloxane ; et
ledit fluide de silicone contient des polymères polydiméthylsiloxane de faible viscosité.

7. Composition transdermique selon l'une quelconque des revendications 1 à 6, dans laquelle ladite insuline est une insuline recombinante humaine préparée dans de l'HCl à 0,01 N, à 50 mg/ml pour obtenir une concentration d'insuline de 20 mg/ml dans la composition d'insuline transdermique stable.

8. Procédé de formulation d'une composition d'insuline transdermique stable pour une administration transdermique d'insuline dans une vasculature dermique comprenant :
la préparation d'un vecteur à partir d'un composant phosphatidylcholine, ledit composant phosphatidylcholine comprenant une phosphatidylcholine enrichie en polyénylphosphatidylcholine et au moins un polyglycol, et
le piégeage de l'insuline dans le vecteur de telle sorte que le vecteur stabilise l'insuline à température ambiante.

9. Procédé selon la revendication 8, dans lequel la préparation dudit vecteur comprend :
la combinaison d'un polyglycol ayant une masse moléculaire de 200 et d'un polyglycol ayant une masse moléculaire de 400 pour former un mélange de polyglycols ;
le prélèvement par raclage dudit composant phosphatidylcholine et l'ajout audit mélange de polyglycols pour former un mélange de phosphatidylcholine ; et
le mélange dudit mélange de phosphatidylcholine jusqu'à ce que ledit mélange de phosphatidylcholine soit clair.

10. Procédé selon la revendication 8 ou 9, dans lequel la préparation dudit vecteur comprend en outre
le chauffage dudit mélange de phosphatidylcholine à 40°C et le brassage de ladite solution chauffée ;
la combinaison d'un agent tensioactif et d'un lubrifiant pour former un fluide ;
l'ajout dudit fluide audit mélange de phosphatidylcholine chauffé et le brassage jusqu'à ce que la solution soit claire ;
l'ajout de méthylparabène audit mélange de phosphatidylcholine et le brassage jusqu'à ce que ledit méthylparabène se dissolve dans ladite solution ;
le chauffage de l'eau à 40°C et l'ajout de ladite eau chauffée lentement à ladite solution ; et
l'arrêt du brassage de ladite solution et l'agitation de ladite solution pour la refroidir à température ambiante.

11. Procédé selon l'une quelconque des revendications 8 à 10, dans lequel ladite insuline est une insuline recombinante humaine préparée dans une solution d'HCl à 0,01 N, à 50 mg/ml et ladite solution d'insuline est mélangée dans ledit vecteur à température ambiante pendant au moins une heure.

12. Procédé selon l'une quelconque des revendications 8 à 11, dans laquelle ladite composition d'insuline transdermique stable contient de l'insuline à une concentration de 20 mg/ml.
